Europäisches Patentamt

European Patent Office

Office européen des brevets

(11) Publication number: **0 261 493**
**A2**

# (12) EUROPEAN PATENT APPLICATION

(21) Application number: 87113092.8

(22) Date of filing: 08.09.87

(51) Int. Cl.4: **G01N 33/53** , G01N 33/68 ,
//G01N33/569

(30) Priority: 23.09.86 IL 80129

(43) Date of publication of application:
**30.03.88 Bulletin  88/13**

(84) Designated Contracting States:
**AT BE CH DE ES FR GB IT LI NL SE**

(71) Applicant: **SAVYON DIAGNOSTICS LTD.**
**1 Jabotinsky Street**
**Ramat-Gan(IL)**

(72) Inventor: **Ben-Michael, Abraham,**
**8, Rehavat Ilan Street, Ramat-Ilan**
**Givat-Shmuel(IL)**

(74) Representative: **Perani, Aurelio et al**
**c/o JACOBACCI-CASETTA & PERANI S.N.C.**
**Via Visconti di Modrone 7**
**I-20122 Milano(IT)**

(54) **Method for the determination of IgM and IgA immunoglobulins and reagents therefor.**

(57) A method is disclosed for determining IgM and IgA antibodies in samples of human serum, which comprises removing IgG antibodies and Rheumatoid Factor from the sample and testing the remaining sample by immunoassay for IgM and IgA antibodies.
Reagents for carrying out the method are also described.

EP 0 261 493 A2

## METHOD FOR THE DETERMINATION OF IgM AND IgA IMMUNOGLOBULINS AND REAGENTS THEREFOR

BACKGROUND OF THE INVENTION

a)The Field of The Invention

The present invention relates to a method for the diagnosis of infectious diseases by the immunoassay determination of IgM and of IgA immunoglobulins in samples of human serum. More particularly, the present invention relates to the determination of IgM and IgA antibodies in samples containing IgG or IgG and Rheumatoid Factor (Rf).

Infectious agents elicit specific antibodies in the blood of the infected person, by means of which the presence of a specific infectious agent can be determined. Such determination is carried out via immunoassays adapted to the identification of such specific antibodies. The identification of the infectious agent and of the stage which the infection has reached in the patient are of paramount importance in order to administer the correct and timely treatment to the patient. It is also important to rule out infection by a certain agent, since different diseases present very similar symptoms. Thus, for instance, mononucleosis caused by the Epstein Bar Virus (EBV) presents symptoms and a pathological picture very similar to that of leukemia. The ruling out of infection by EBV is therefore very important in the efficient and quick treatment of the leukemia patient.

There are three different main types of antibodies, called immunoglobulins, namely IgM, IgG and IgA which can be identified in the human serum. These antibodies can be roughly divided by chronological appearance in the serum following an infection by an infectious agent, since IgM indicates a primary infection and IgG a continued infection. The role of IgA is not altogether clear, but it is believed to be indicative of a chronic infection. While reference will be made hereinafter mainly to IgM, for the sake of brevity, the same problems and solutions apply to IgA as well. It should be understood that it is not necessary to carry out the determination of IgM and IgA at the same time, and that, according to the method of the invention, it is possible - if desired - to determine only one of IgM and IgA, according to the specific diagnostic requirements.

It is therefore clear that a separate and clear identification of IgG and IgM antibodies is important, in order to obtain a clear picture of the disease and the stage in which it is found. The mere indication that some antibodies specific to a certain disease are present in the serum is not satisfactory for the purpose of prescribing the most appropriate treatment. This means that it is not sufficient to determine IgG alone or IgM alone, in order to obtain a full serological picture of the infection, but both IgG and IgM must be tested, and further it is recommendable to determine IgA as well. It is likewise clear that false positive and false negative results of immunoassays may lead to a wrong treatment and may be dangerous to the patient. Unfortunately, IgM is very difficult to determine in the presence of IgG, since IgG is a much smaller molecule which has a masking effect on other globulins due to its very high concentration (up to 90% of the total immunoglobulins content) and to its high affinity to the appropriate antigen. Moreover, an auto-immune IgM anti-IgG is known to bind to IgG in the serum -the so-called rheumatoid factor. Titers as high as 1:1280 of the Rf IgM can be found in human serum, which give raise to false-positive IgM responses in immunoassays.

b) The Prior Art

The art has constantly been looking for ways to obviate the said problems for an effective determination of IgM antibodies. Two methods are widely used for increasing the sensitivity of immunoassays towards IgM, or for permitting its detection, both which involve the treatment of the tested serum. The first method comprises treating the serum with Protein A which absorbs both IgG and Rf and permits their separation from the serum. This method, however, presents several severe drawbacks. The diagnostically useful range of action of Protein A is very narrow and required very fine calibration since the use of excess Protein A results in the removal of a considerable part of the IgM that it is desired to determine, giving raise to false-negative responses, and too low amounts do not remove all the IgG, leading to false-negative results - and the Rf, leading to false-positive results. Furthermore, Protein A is very expensive - present costs being as high as US$ 15 per mg - and is also a dangerous irritant. Protein A, besides the above disadvantages, is also a very unstable reagent, whose useful shelf life is very short.

Another method which is employed to overcome the false-positive response due to Rf involves the use of aggregated IgG. In this method the Rf IgM is shifted from the serum IgG to the aggregated IgG added to the serum, and then precipitated. This method, however, does not solve the probles of masking by IgG and is further very sensitive to aggregated IgG concentration thus requiring delicate calibration. A considerable portion of IgM from the serum can also be removed when operating with this method, together with the Rf, IgM, giving again raise to false negative responses.

The above disadvantages and laboratory difficulties result, in practice, in that IgM determination is not carried out on a large scale and as a routine test, both because the very high cost of the said reagents, and because of the delicate and difficult procedures necessary for their use. It is therefore clear that it is a very desirable goal to provide means for permitting to carry out the determination of IgM and IgA antibodies of infectious diseases as a routine test and to reduce the danger of false positive and false negative results, which determination can be carried as it is presently more easily performed for IgG.

## SUMMARY OF THE INVENTION

It is an object of the present invention to provide a method of which the determination of IgM and IgA antibodies in samples of human serum can be carried out with accuracy, in a simple manner and at low costs.

It is a further object of the invention to provide a reagent for carrying out the said method and tests.

It has also been surprisingly found, and this is still another object of the invention, that it is possible to carry out an effective and meaningful determination of IgM and IgA antibodies in spite of the fact that ameasurable amount of IgM and IgA is removed from the serum, together with the IgG and the Rf, and that in spite this removal of the tested globulins, the expression of IgM and IgA in IPA is increased. As compared, e.g., to the Protein A method, the method of this invention has the considerable advantage of presenting a very wide effective reagent concentration range, with respect to IgG and Rf removal. Surprisingly, in this range the concentratons of IgM and IgA are either unaffected or slightly reduced (up to 20-40% maximum). In contrast, if the Protein A concentration exceeds its narrow effective range, both IgM and IgA concentrations drop to low values and may even reach undetectable values. This drawback causes Protein A to be a much less practical method than the method of the invention.

## DETAILED DESCRIPTION OF THE INVENTION

The method for the determination of IgM and of IgA antibodies in samples of blood serum according to the invention is characterized in that the IgG antibody and the Rheumatoid Factor present in the serum are substantially removed therefrom by precipitating them with a zinc ion and by separating the remaining liquid from the said precipitate and testing the said remaining liquid by immunoassay for IgM and IgA antibodies.

According to a preferred embodiment of the invention, up to 40% of the IgM and up to 20% of the IgA originally present in the serum can be precipitated and removed therefrom, together with the IgG and the Rf.

A preferred embodiment of the invention is characterized by the steps of:

a) preparing a diluted solution of the serum to be tested, in a buffer;

b) adding a solution of one or more zinc salt(s) to the diluted serum, under moderate stirring;

c) incubating the so obtained solution for at least about 5 minutes;

d) centrifuging the solution of step (c);

e) separating the liquid from the precipitate obtained in step (d); and

f) testing the said liquid for the presence of IgM and IgA antibodies.

Preferably, steps (b) through (f) are carried out at a temperature that does not exceed 30°C, more preferably at a temperature of about 0°-4°C.

The incubation step (c) can be carried out for long periods, if desired, and is typically carried out for one hour. The minimal period of 5 minutes, however, should be allowed for obtaining the precipitation of at least the major part of the IgG from the serum.

The buffer may be any buffer whose components do not interact with the zinc ion, such as buffers containing chelating agents or anions of insoluble zinc salts, which will remove the zinc ion from the solution. Thus buffers such as the phosphate or citrate buffers are unsuitable for carrying out the method of the invention.

The buffering component of the buffer is preferably selected from among tris(hydroxymethyl)-aminomethane (TRIS), N,N-bis[2-hydroxyethyl]-2-aminoethane-sulfonic acid (BES), N,N-bis[2-hydroxyethyl]-glycine (BICINE), N-2-hydroxyethylpiperazine-N'-2-ethanesulfonic acid (HEPES), 2-[N-morpholino]-ethanesulfonic acid (MES), 3-[N-morpholino] ethanesulfonic acid (MOPS), N-tris[hydroxymethyl]methyl-2-aminoethanesulfonic acid (TES) and N-tris[hydroxymethyl] methylglycine (TRICINE).

More preferably the buffer employed in each step is tris buffer saline (TBS), the concentration of the zinc ion in the final solution is between 0.005M and 0.2M, preferably about 0.05M.

The final dilution of the serum may vary in a very wide range, e.g., from 1:4 to 1:1000, and is dictated by working requirements. For a practical use it has been found that a dilution of 1:20 is convenient although, as stated, not limitative.

The zinc salt that provides the zinc ion can be any organic or inorganic salt of zinc, provided that it is not an unsoluble salt and provided that the anion does not behave as a chelating agent. Thus, for instance, citrate or tartarate salts of zinc cannot be employed in the method of the invention. Examples of suitable organic salts are the diglycinate, glutaminate, malate, aconitate, glycolate, tricarballylate, succinate, lactate and taurate salts of zinc, and examples of suitable inorganic salts are the chloride, sulphate and nitrate salts of zinc. The most preferred salt, to be used as a zinc source in the method of the invention, is the diglycinate salt of zinc. Since the zinc ion is the effective and specific precipitating agent of IgG, more than one different salt of zinc can be employed in the method of the invention. Thus, for instance, different salts can be combined having different dissociation constants, to provide differently timed release of the zinc ion into the solution.

Zinc salts as defined above, whenever employed as a zinc source to be used in IgM and IgA determinations according to the method of the invention, also form part of the present invention.

The above and other advantages and characteristics of the invention will be better understood through the following illustrative and non-limitative description of preferred embodiments.

## Example 1

### Preparation of the reagent zinc diglycinate

To 500 ml of hot (80°C) distilled water there were added 18.75 gr glycine (0.25 moles). Into this solution there were slowly dissolved 8.137 gr of zinc oxide (0.1 moles), care being taken that each added drop is been completely dissolved, keeping a clear solution, before adding the next drops. After dissolution of the zinc oxide was completed, 87.8 gr of zinc acetate (0.4 moles) were further dissolved into the solution and the total content of the resulting solution was brought to 1 liter by adding distilled water under stirring. The 1 liter solution so obtained was kept under stirring overnight at room temperature. The resulting solution contained a 0.5M solution of the zinc diglycinate reagent.

## Example 2

### Removal of IgG and Rf from Human Serum and Preparation of Serum for IgM and IgA determination by Immunoassay.

The serum to be tested is diluted to a 1:10 dilution in Tris Buffer Saline (TBS), by adding 180 μl TBS to 20 μl of patient's serum. The test tube is placed in a container filled with ground ice. To the test tube are further added 200 μl of the zinc diglycinate solution of Example 1, thus obtaining a serum with a 1:20 dilution. After addition of the zinc diglycinate solution, the contents of the test tube are gently mixed on a Vortex for 30 seconds, after which the test tube is kept in a refrigerator at 4°C for one hour.

At the end of the one hour period, the test tube is passed to a cooled centrifuge (4°C) and is centrifuged at 3000 rpm for 15 minutes. At the end of this period a precipitate is present in the test tube. The upper liquid is withdrawn and passed into another test tube, adapted for the immunoassay for determining immunoglobulins.

### Example 3

A sample of human serum was tested by the Immunoperoxidase Assay (IPA) for the presence of antibodies to Chlamydia trachomatis (IPAzyme Chlamydia, manufactured by Savyon Diagnostics Limited). The test gave as a result a titer of 1:512 for IgG and no presence of IgM was determined in the sample.

The sample was treated as in Example 2 and was then tested again by the same test. No preference of IgG was detectable, while a 1:128 titer of IgM and a 1:64 titer of IgA were found in the test.

About 20% of the IgM and about 15% of the IgA originally present in the sample were removed through the treatment with zinc diglycinate.

### Example 4 (Comparative)

Example 3 was repeated, using Protein A as the precipitation reagent. Calibration was carried out as described hereinafter in Example 10. The final results showed a 1:128 titer of IgM and a 1:16 titer of IgA.

### Example 5

Example 3 was repeated with a serum found positive to IgG antibody to EBV. The results were as in Example 3, with a 1:256 titer of IgM.

### Example 6

Example 3 was repeated with a serum found positive to IgG antibody to Cytomegalovirus. The results were as in Example 3, with a 1:1024 titer of IgM.

### Example 7

A serum of a patient was found to be positive to IgG (titer 1:512) and IgM (titer 1:1024) specific to Chlamydia trachomatis. The serum was treated as in Example 2 and was found to contain no detectable IgG and to be negative to IgM, pointing to a false-positive response due to a high level of Rf in the serum. The patient whose serum was tested in this test was therefore positive to Chlamydia trachomatis, since IgG was present in the serum, but no current infection was present, since no IgM was detectable.

### Example 8

A sample of human serum was tested by IPA for the presence of antibodies to EBV (IPAzyme EBV, manufactured by Savyon Diagnostics Limited). The test gave as a result a titer of 1:512 for IgG and no presence of IgM was determined in the sample.

The sample was treated as in Example 2 and was then tested again by the same test. No presence of IgG or IgA was detectable, while a 1:256 titer of IgM was found in the test.

### Example 9

Calibration of the zinc ion concentration.

A sample of serum was treated at different $Zn^{++}$ concentrations, as in Example 2. IgG and IgM were determined by the Immunoperoxidase Assay (IPA). The results of these tests are shown in Table 1 which shows the percent remaining immunoglobulin, following treatment as in Example 2, as compared to the concentration in the untreated serum specimen.

## Table 1

| Zn$^{++}$ Conc. (mmole/lit) | IgG (%) | IgM (%) |
|---|---|---|
| 0 | 100 | N.A. |
| 0.1 | 100 | N.A. |
| 0.5 | 100 | N.A. |
| 1.0 | 75-80 | N.A. |
| 2.5 | 40-50 | N.A. |
| 5.0 | 10-20 | N.A. |
| 10.0 | 0-10 | 80-100 |
| 20.0 | 0 | 80-100 |
| 30.0 | 0 | 80-100 |
| 40.0 | 0 | 80-100 |
| 50.0 | 0 | 80-100 |
| 60.0 | 0 | 80-100 |
| 75.0 | 0 | 80-100 |
| 100.0 | 0 | 80-100 |
| 200.0 | 0 | 80-100 |

N.A. - Not Applicable. At the indicated Zn$^{++}$ concentration the IPA results for IgM are unreliable and non-reproducible.

As it can be appreciated from inspection of the results in Table 1, the range in which reliable and reproducible IPA results can be obtained is very wide, namely 10 to 200 mmole/liter, so that no accurate and repeated calibration is required when operating according to the invention.

### Example 10 (Comparative)

#### Calibration of Protein A - Bacterial Absorbent (SpA)

A calibration of SpA (prepared from Staphyloccus aureus Cowan 1 strain) was carried out, similarly to the calibration carried out in Example 9 above, with the appropriate changes.

A SpA lyophilized material was reconstituted with distilled water to obtain a 10% v/v suspension and well vortexed to complete reconstitution. The suspension was then divided between centrifuge tubes and centrifuged for 25 minutes at 2000 g. The supernatant was discarded at the end of centrifugation. The pellets found in the test tubes were re-suspended to the original volume with phosphate buffer saline. All the suspended material was then collected into one pool and $^{NaNO_3}$ was added to a final concentration of 1 mg/ml (0.1% w/v) to prevent growth of bacterial.

The following volumes of the suspension were then pipetted into a series of test tubes: 2.0 ml, 1.0 ml, 0.5 ml and 0.25 ml. Each series is for testing one serum specimen. The test tubes with the SpA suspension were centrifuged for 25 minutes at 2000 g and the supernatant discarded from each tube. To the pellets in each tube were added 100 microliters phosphate buffer saline. Test tubes not immediately used can be stored at -20°C.

To each of the 5 test tubes of each series were then added 25 microliters of the serum specimen and all test tubes were then vortexed at the same time, to avoid variability of the absorption time. After the contents of the test tubes were thoroughly mixed, the tubes were incubated in a 37°C water bath with a precise temperature control, for exactly 10 minutes. After the end of this period, the tubes were centrifuged at room temperature for 25 minutes at 2000 g, thereby terminating the IgG/Rf absorption step.

The supernatant from each test tube was collected and passed to a new test tube. The test tube containing the pellets were discarded. The supernatant were tested by IPA for IgG and IgM. Since the absorption potency of SpA is not constant, the above calibration procedure must be carried out at least once every 4-5 runs. The results obtained are shown in Table 2 below.

## Table 2

| SpA quantity (ml) (10% suspension) | IgG (%) | IgM (%) |
|---|---|---|
| 2.0 | 0 | 0 |
| 1.0 | 0 | 70-100 |
| 0.5 | 40-50 | N.A. |
| 0.25 | 80-100 | N.A. |
| 0 | 100 | N.A. |

As it can be appreciated from the above results, the applicable range of Protein A is very narrow and, also together with the inconstant absorption potency, very much reduces the reproducibility of the results, unless complicated and lengthy calibration procedures are carried out very often. Furthermore, an even small deviation in SpA effective concentration - which may also be due to SpA deterioration with time - causes extreme changes in IgM and IgG removal and may lead to incorrect results.

As it will be apparent to the person skilled in the art, the method of the invention permits to remove the masking effect of IgG and false-positive effects due to Rf, thereby permitting to determine the presence of IgM and IgA antibodies. The method may cause a reduction of the content of IgM and IgA, some of which can be removed from the serum together with the IgG and Rf. However, when operating according to the method of the invention, the amount of IgM removed does not exceed 40% of the original content, and that of IgA does not exceed 20% of the original content, while IgG and Rf are removed in sufficient amounts as to make them undetectable by commonly employed assays. Therefore, the expression of IgM and IgA in the serum as obtained by IPA, after removal of the masking effect of IgG, is considerably increased, which permits their easy and positive determination. It is, of course, possible to remove amounts lower than the above-specified maximal removed amounts of IgM and IgA, by employing lower reagent concentrations, thereby leaving higher contents of IgG and Rf in the sample tested. The amounts removed depend on the specific requirements of the test that it is desired to carry out, as it will be apparent to the man of the art.

Another prominent advantage of the method of the invention is that the reagent employed and the treatment of the serum are simple and cheap. The material required for one test with zinc diglycinate is estimated to be about 40,000 times cheaper than that required for a similar test employing Protein A ($1.43 per test using Protein A/ $0.00004 per test according to the invention). It is clear that this impressive difference in cost can permit to carry out IgM and IgA determinations in a routine way. The zinc salts can be conveniently desk stored as solutions ready to be used while, in comparison, Protein A can be shipped and stored only in a dry (lyophylized) form and the solution must be prepared shortly before use. The low cost and the simple procedures provided by the invention, together with the long shelf life of the zinc salts as compared with the very short shelf life of, e.g., Protein A, as well as the high reliability of the method for the determination of IgM and IgA, are such that can permit a widespread use of this very important diagnostic route, which has been so far limited as explained above.

The above description and examples have been given for the purpose of illustration and are not intended to be limitative. Many variations can be effected in the various quantities, procedures and reagents, without departing from the method of the invention or exceeding its scope.

## Claims

1. A method for the determination of IgM and IgA antibodies in samples of blood serum, characterized in that the IgG antibody and the Rheumatoid Factor present in the serum are substantially removed therefrom by precipitating them with a zinc ion and by separating the remaining liquid from the said precipitate and testing the said remaining liquid by immunoassay for IgM and IgA antibodies.

2. A method according to claim 1, characterized in that up to 40% of the IgM and up to 20% of the IgA originally present in the serum are precipitated and removed therefrom, together with the IgG and the Rf.

3. A method according to claim 1 or 2, characterized by the steps of:

    a) preparing a diluted solution of the serum to be tested, in a buffer;

    b) adding a solution of one or more zinc salt(s) to the diluted serum;

    c) incubating the so obtained solution for at least about 5 minutes;

    d) centrifuging the solution of step (c);

    e) separating the liquid from the precipitate obtained in step (d); and

    f) testing the said liquid for the presence of IgM and IgA antibodies.

4. A method according to claim 3, characterized in that steps (b) through (f) are carried out at a solution temperature lower than about 30°C.

5. A method according to claim 4, wherein the temperature is between 0° and 10°C, preferably about 0° - 4°C.

6. A method according to any one of the preceding claims, characterized in that the buffering component of the buffer is selected from among tris(hydroxymethyl)aminomethane (TRIS), N,N-bis[2-hydroxyethyl]-2-aminoethane-sulfonic acid (BES), N,N-bis[2-hydroxyethyl]-glycine (BICINE), N-2-hydroxyethylpiperazine-N'-2-ethanesulfonic acid (HEPES), 2-[N-morpholino]ethanesulfonic acid (MES), 3-[N-morpholino] ethanesulfonic acid (MOPS), N-tris[hydroxymethyl]methyl-2-aminoethanesulfonic acid (TES) and N-tris[hydroxymethyl] methylglycine (TRICINE).

7. A method according to any one of the preceding claims, wherein the concentration of the zinc ion in the final solution is between 0.005M and 0.2M, preferably about 0.05M.

8. A method according to any one of the preceding claims characterized in that the zinc salt is selected from among the diglycinate, glutaminate, malate, aconitate, glycolate, tricarballylate, succinate, lactate taurate, chloride, sulphate and nitrate salts of zinc.

9. A method according to claim 8, wherein the zinc salt is zinc diglycinate.

10. A precipitating agent to be used in IgM and/or IgA determination, characterized in that it is a zinc salt.

11. The precipitating agent of claim 10, characterized in that it is zinc diglycinate.

12. A method for the determination of IgM and IgA antibodies essentially as described and illustrated, with particular reference to the examples.